# EUROPEAN PATENT APPLICATION

(11) **EP 4 431 106 A2**
(43) Date of publication of application: **18.09.2024**
(21) Application number: 22893239.8
(22) Date of filing: 10.11.2022
(51) Int. Cl.: A61K 36/35, A61K 31/7004, A61P 21/00, A23L 33/105, A23L 33/125

(54) **PHARMACEUTICAL COMPOSITION FOR PREVENTING OR TREATING MUSCLE DISEASES**

(30) Priority: 12.11.2021 KR 20210155705
(71) Applicant: Kosa Bio Inc., Namyangju-si, Gyeonggi-do 12106 (KR)
(72) Inventor: KONG, Hyunseok, Seoul 03730 (KR); KIM, Jiyeon, Seoul 02439 (KR); HONG, Seonhwa, Namyangju-si, Gyeonggi-do 12056 (KR); KIM, Jongsu, Seongnam-si, Gyeonggi-do 13508 (KR)
(74) Representative: Dehns
(86) International application number: PCT/KR2022/017671
(87) International publication number: WO 2023/085820

(57) **Abstract**

The present disclosure relates to a pharmaceutical composition for preventing or treating muscle diseases. Particularly, an elderberry extract or a monosaccharide- and amino acid-coupled compound, according to the present disclosure exhibits the effects of inhibiting muscle cell apoptosis, inhibiting the expression of inflammatory cytokines, promoting the secretion of testosterone, inhibiting the expression of muscle atrophy markers and myostatin and causing muscle to recover in sarcopenia animal models, and thus can be effectively used in the treatment of muscle diseases.

## Description

### Technical Field

The present disclosure relates to a pharmaceutical composition for the prevention or treatment of muscle diseases.

### Background Art

Muscle is the most abundant tissue in the human body, and the maintenance of adequate muscle mass is essential for functional capability in the human body and required for preventing metabolic diseases. The size of muscle is regulated by intracellular signaling processes that induce anabolism or catabolism within the muscle. When signaling responses that induce more synthesis than degradation of muscle proteins occur, there is an increase in muscle protein synthesis, leading to hypertrophy or an increase in the number of muscle fibers (hyperplasia).

Moreover, muscle also enhances bone density by promoting the influx of calcium. However, as the body ages, its components change, resulting in the redistribution of body fat and body protein. After the age of 50, the rate of protein synthesis within muscle cells slows compared to the rate of degradation, resulting in a rapid degeneration of muscles and consequently exposing individuals to diseases caused by muscle loss.

One such disease caused by muscle loss is sarcopenia, where the body's muscle mass decreases to about 13-24% of body mass, showing not only a reduction in muscle mass but also in protein content, fiber diameter, muscle strength, and fatigue resistance. Sarcopenia can result from various causes such as sepsis, cancer, renal failure, excessive glucocorticoids, nerve removal, muscle disuse, and aging, typically due to the gradual decrease in quantity and quality of skeletal muscle or inappropriate dietary-induced weight loss including fat and body fat components.

Sarcopenia arises from an imbalance between protein synthesis and degradation. If present, sarcopenia significantly reduces the amount of physical activity, lowering the quality of life and making it easier to sustain injuries during daily activities. Moreover, excessive exercise can also induce muscle fatigue and damage, with the consequent onset of pain and temporary loss of motor skills.

In relation, Korean Patent Publication No. 10-2022-0113912 discusses a composition including a castor oil plant extract as an active ingredient for the prevention, alleviation, or treatment of muscle diseases. This extract is disclosed to increase the size of muscle cells and improve exercise performance, thereby preventing, improving, or treating muscle diseases without side effects.

### Disclosure of Invention

### Technical Problem

The present disclosure aims to provide a use of elderberry extract for the prevention, alleviation, or treatment of muscle diseases.

The present disclosure is also to provide a use of elderberry extract for muscle strengthening.

Furthermore, the present disclosure is to provide a use of a monosaccharide and amino acid conjugate compound, or a pharmaceutically acceptable salt thereof for the prevention, alleviation, or treatment of muscle diseases.

Moreover, the present disclosure is to provide a use of a monosaccharide and amino acid conjugate compound, or a pharmaceutically acceptable salt thereof for muscle strengthening.

### Solution to Problem

To achieve the aims, an aspect of the present disclosure provides a pharmaceutical composition including elderberry extract as an active ingredient for the prevention or treatment of muscle diseases.

Also, another aspect of the present disclosure provides a health functional food including an elderberry extract as an active ingredient for the prevention or alleviation of muscle diseases.

Additional another aspect of the present disclosure provides a health functional food including an elderberry extract as an active ingredient for muscle strengthening.

Yet another aspect of the present disclosure provides a method for the prevention, alleviation, or treatment of muscle diseases, the method including a step of administering elderberry extract to a subject.

Moreover, another aspect of the present disclosure provides a use of an elderberry extract in the preparation of a medicament for the prevention, alleviation, or treatment of muscle diseases.

Another aspect of the present disclosure provides a pharmaceutical composition including a monosaccharide and amino acid conjugate compound, or a pharmaceutically acceptable salt thereof as an active ingredient for the prevention or treatment of muscle diseases,

Another aspect of the present disclosure provides a health functional food including a monosaccharide and amino acid conjugate compound, or a pharmaceutically acceptable salt thereof as an active ingredient for the prevention or alleviation of muscle diseases.

Another aspect of the present disclosure provides a health functional food including a monosaccharide and amino acid conjugate compound, or a pharmaceutically acceptable salt thereof as an active ingredient for muscle strengthening.

Another aspect of the present disclosure provides a method for the prevention, alleviation, or treatment of muscle diseases, the method including a step of administering a monosaccharide and amino acid conjugate compound, or a pharmaceutically acceptable salt thereof to a subject.

Another aspect of the present disclosure provides a use of including a monosaccharide and amino acid conjugate compound, or a pharmaceutically acceptable salt thereof in the preparation of a medicament for the prevention, alleviation, or treatment of muscle diseases.

### Advantageous Effects of Invention

Particularly, an elderberry extract or a monosaccharide- and amino acid-coupled compound, according to the present disclosure exhibits the effects of inhibiting muscle cell apoptosis, inhibiting the expression of inflammatory cytokines, promoting the secretion of testosterone, inhibiting the expression of muscle atrophy markers and myostatin and causing muscle to recover in sarcopenia animal models, and thus can be effectively used in the treatment of muscle diseases.

### Brief Description of Drawings

FIG. 1 shows microscopic observation of the morphology of myoblasts before (A) and after (B) differentiation into myotubes in an embodiment of the present disclosure.
FIG. 2 shows the microscopic observation of the morphology of myotubes before (A) and after (B) treatment with dexamethasone in an embodiment of the present disclosure.
FIG. 3 shows graphs of the effect of suppressing myocyte apoptosis by hot water extract of elderberry (A), 50% ethanol extract of elderberry (B), or 100% ethanol extract of elderberry (C) in an embodiment of the present disclosure.
FIG. 4 shows graphs of the effect of suppressing myocyte apoptosis by FL (A), FV (B), or FI (C) in an embodiment of the present disclosure.
FIG. 5 shows graphs of the effect of suppressing muscle cell apoptosis by various amino acids (leucine, valine, arginine, tyrosine, methionine, or phenylalanine) in an embodiment of the present disclosure.
FIG. 6 shows graphs of the effect of suppressing muscle cell apoptosis by various amino acids (isoleucine, tryptophan, glycine, lysine, or threonine) in an embodiment of the present disclosure.
FIG. 7 shows graphs of the effect of inhibiting TNFα expression by hot water extract of elderberry (A), 50% ethanol extract of elderberry (B), or 100% ethanol extract of elderberry (C) in an embodiment of the present disclosure.
FIG. 8 shows graphs of the effect of inhibiting TNFα expression by FL (A), FV (B), or FI (C) in an embodiment of the present disclosure.
FIG. 9 shows graphs of the effect of promoting testosterone secretion by hot water extract of elderberry (A) or FL (B) in an embodiment of the present disclosure.
FIG. 10 shows graphs of the effect of inhibiting MuRF-1 gene expression by hot water extract of elderberry (A), 50% ethanol extract of elderberry (B), or 100% ethanol extract of elderberry (C) in an embodiment of the present disclosure.
FIG. 11 shows graphs of the effect of inhibiting atrogin-1 gene expression by hot water extract of elderberry (A), 50% ethanol extract of elderberry (B), or 100% ethanol extract of elderberry (C) in an embodiment of the present disclosure.
FIG. 12 shows graphs of the effect of inhibiting myostatin gene expression by hot water extract of elderberry (A), 50% ethanol extract of elderberry (B), or 100% ethanol extract of elderberry (C) in an embodiment of the present disclosure.
FIG. 13 shows graphs of the effect of inhibiting MuRF-1 gene expression by FL (A), FV (B), or FI (C) in an embodiment of the present disclosure.
FIG. 14 shows graphs of the effect of inhibiting atrogin-1 gene expression by FL (A), FV (B), or FI (C) in an embodiment of the present disclosure.

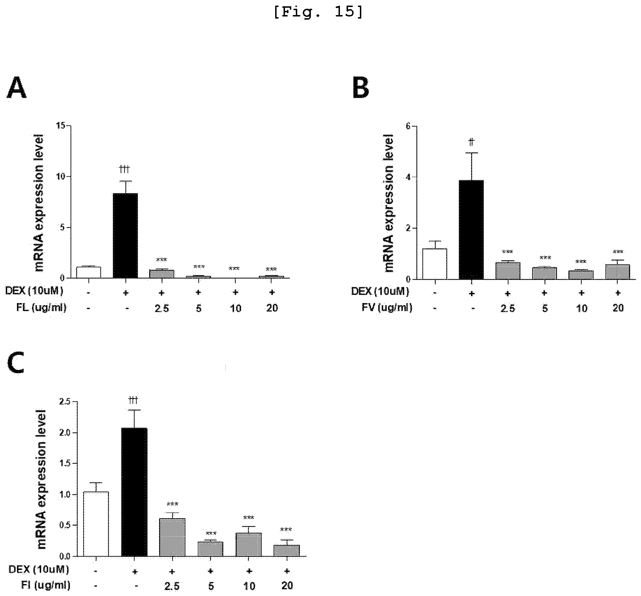

FIG. 15 is a graph showing the effect of inhibiting myostatin gene expression by FL (A), FV (B), or FI (C) in an embodiment of the present disclosure.

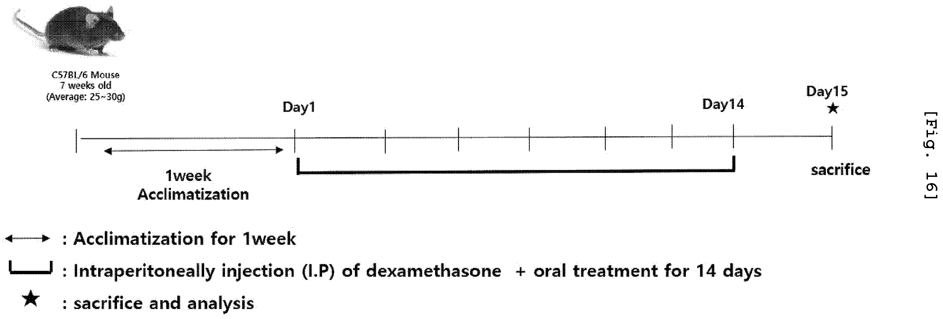

FIG. 16 is a schematic diagram showing an animal experiment plan using a sarcopenia animal model in an embodiment of the present disclosure.

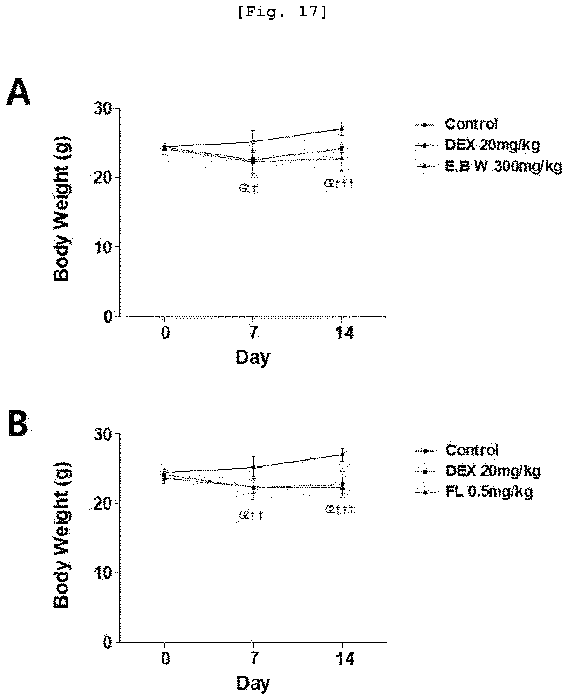

FIG. 17 shows plots of the results of body weight changes in a sarcopenia animal model treated with hot water extract of elderberry (A) or FL (B) in an embodiment of the present disclosure.

### Best Mode for Carrying out the Invention

Below, a detailed description will be given of the present disclosure.

The present disclosure provides a pharmaceutical composition including an elderberry extract as an active ingredient for the prevention or treatment of muscle diseases.

The term "elderberry", used herein, refers to a berry fruit that is dark purple to black in color and is commonly called "black elder" as it usually fruits in the fall. In North America, elderberry extract is also referred to as "sambucol". Elderberries are known to be rich in vitamins A, B, C, and anthocyanins, and have been reported to be effective in preventing colds and boosting immunity.

The elderberry extract can be produced by a preparation method that includes the following steps of:
1) adding an extraction solvent to elderberries to obtain an extract;
2) filtering the extract from step 1); and
3) concentrating the filtrate from step 2) under reduced pressure and then drying the concentrate.

Also, the extraction solvent may be water, alcohol, or a mixture thereof. The alcohol may be a lower alcohol of C1 to C2, specifically, ethanol, methanol, or rectified spirit. The extraction solvent can be added in an amount of 1 to 100 times, 1 to 70 times, 1 to 50 times, 1 to 30 times, or 1 to 15 times the weight of the elderberries. If alcohol is used as the extraction solvent, it may be 10 to 100%, 20 to 100%, 30 to 100%, or 40 to 100% alcohol.

The extraction method can be shaking extraction, maceration, reflux extraction, or ultrasonic extraction. The extraction may be conducted for 1 to 20 hours, 2 to 20 hours, 1 to 10 hours, 2 to 10 hours, 1 to 5 hours, or 2 to 5 hours. The extraction may be repeated more than once.

Moreover, the concentration under reduced pressure in step 3) may be performed using a vacuum rotary evaporator or a vacuum concentrator. The drying may be vacuum drying, reduced pressure drying, boiling drying, spray drying, or freeze-drying, specifically freeze-drying.

The muscle disease may be a disease caused by muscle reduction. In another aspect, the muscle disease may be a progressive disorder that includes the loss of walking ability, weakening of respiratory muscle strength, and weakening of heart function due to the gradual decrease in muscle strength. Additionally, the muscle disease may be a congenital or acquired disorder. For example, diseases caused by muscle reduction may include atony, muscular atrophy, muscular dystrophy, muscle degeneration, myotonia, muscular dystrophy, amyotrophic lateral sclerosis (ALS), myasthenia, cachexia, or sarcopenia.

The pharmaceutical composition according to the present disclosure may include the monosaccharide and amino acid conjugate compound or pharmaceutically acceptable thereof as an active ingredient in an amount of 10 to 95 % by weight based on the total weight thereof. The pharmaceutical composition of the present disclosure may include, in addition to the active ingredient, one or more effective ingredients having identical or similar function to the active ingredient.

The pharmaceutical composition of the present disclosure may include carriers, diluents, excipients or mixtures thereof generally used in biological preparations. The pharmaceutically acceptable carrier may be any carrier suitable for delivering the composition in human body. In detail, the carrier may be saline, sterilized water, Ringer's solution, dextrose solution, maltodextrin solution, glycerol, ethanol, or a mixture thereof, as exemplified by the compounds described in Merck Index, 13th ed., Merck & Co. Inc. If necessary, a general additive such as antioxidant, buffer, bacteriostatic agent and others may be additionally added.

When formulating the composition, commonly used diluents or excipients, such as fillers, extenders, binders, wetting agents, disintegrants, and surfactants, can be added.

The composition of the present disclosure can be formulated as oral preparations or non-oral preparations. Oral formulations can include solid formulations and liquid formulations. The solid formulations may be tablets, pills, powders, granules, capsules or troches. These solid formulations are prepared by mixing the composition with one or more suitable excipients. These excipients may include starch, calcium carbonate, sucrose, lactose, gelatin, or a mixture thereof. Furthermore, the solid preparation may contain lubricants such as magnesium stearate and talc. The liquid formulations can be suspensions, solutions, emulsions or syrups, and the above-mentioned liquid formulations can contain various excipients such as wetting agents, sweeteners, aromatics and preservatives.

Formulations for non-oral administration can include injections, suppositories, powders for respiratory inhalation, aerosols for spray, powders and creams. The injection can include sterilized aqueous solutions, non-aqueous solvents, suspension solvent, emulsions, and the like. In this regard, examples of the non-aqueous solvent or suspension solvent include propylene glycol, polyethylene glycol, vegetable oil such as olive oil, injectable ester such as ethyl oleate, and the like.

Additionally, the present disclosure provides a health functional food containing elderberry extract as an active ingredient for the prevention or alleviation of muscle diseases.

The elderberry extract included in the health functional food according to the present disclosure can have the characteristics as described above. The muscle diseases may also have the characteristics as previously described.

The elderberry extract of the present disclosure can be added directly to food or used in conjunction with other food ingredients. The content of the active ingredient added may be determined according to the purpose and typically may constitute 0.01 to 90 weight parts based on the total weight of the health functional food.

No particular limitations are limited to the form and type of the health functional food. Specifically, the health functional food may be in the form of tablets, capsules, powders, granules, liquids, or pills. The health functional food may include various additives such as flavorings, sweeteners, or natural carbohydrates. The sweeteners may be natural or synthetic; examples of natural sweeteners include thaumatin, stevia extract, and others, while examples of synthetic sweeteners include saccharin, aspartame, and others. Additionally, the natural carbohydrates may include monosaccharides, disaccharides, polysaccharides, oligosaccharides, sugar alcohols, and others.

The health functional food of the present disclosure, in addition to the aforementioned additives, may further include nutrients, vitamins, electrolytes, flavor enhancers, colorants, pectic acid and its salts, alginic acid and its salts, organic acids, protective colloidal thickening agents, pH adjusters, stabilizers, preservatives, glycerin, alcohol, and others. These components may be used independently or in combination. The ratio of these additives may be selected within the range of 0.01 to 0.1 weight parts per 100 weight parts of the composition of the present disclosure.

Furthermore, the present disclosure provides a health functional food containing elderberry extract as an active ingredient for muscle strengthening.

The elderberry extract included in the health functional food according to the present disclosure can have the characteristics as previously described. The health functional food itself may also have the same characteristics described in the foregoing.

Additionally, the present disclosure provides a method for the prevention, alleviation, or treatment of muscle diseases, the method including a step of administering an elderberry extract to a subject.

The elderberry extract used in this method and muscle diseases can have the characteristics as described above.

The subject may be a mammal, specifically a human.

Depending on a desired manner, the administration may be oral or non-oral, where non-oral administration may include intraperitoneal, rectal, subcutaneous, intravenous, intramuscular, or intrathoracic injection methods.

The administration should be in a pharmacologically effective amount, which can vary depending on the type of disorder, its severity, the activity of the drug, patient sensitivity to the medication, timing of administration, route of administration, duration of treatment, and any concurrently used medications. However, for desired effects, the amount of the active ingredient included in the administration can range from 0.0001 to 1,000 mg/kg, specifically 0.001 to 500 mg/kg. The administration may occur once or several times a day.

The administration can be performed alone or in combination with other therapeutic agents. When combined, the administration may be sequential or simultaneous.

Furthermore, the present disclosure provides a use of an elderberry extract in the preparation of medicaments for the prevention, alleviation, or treatment of muscle diseases.

The elderberry extract used in the preparation of these medicaments can have the characteristics as described above. The muscle diseases can also have the same characteristics described in the foregoing.

Additionally, the present disclosure provides a pharmaceutical composition including a monosaccharide and amino acid conjugate compound, or a pharmaceutically acceptable salt thereof as an active ingredient for the prevention or treatment of muscle diseases.

As used herein, the term "monosaccharide" refers to the most basic units of carbohydrates, also known as simple sugars. Generally, monosaccharides are colorless, water-soluble, crystalline solids that include glucose, fructose, galactose, and so on. In one embodiment of the present disclosure, the monosaccharide could be fructose. "Fructose" is a monosaccharide containing six carbon atoms and a ketose possessing a ketone group, represented chemically as C₆H₁₂O₆. Fructose serves as an intermediate in the breakdown of glucose and the synthesis of glycogen in biological sugar metabolism.

The term "amino acid", as used herein, refers to the basic building units of proteins in organisms, which chemically contain both a basic amino group (-NH₂) and an acidic carboxyl group (-COOH). Typically, when proteins are hydrolyzed by acids or enzymes such as pepsin or trypsin, they break down into various types of amino acids. These amino acids are then absorbed into the body, rearranged, and synthesized into the proteins that make up the body. Amino acids are classified into non-essential and essential amino acids.

The amino acids may include all types known in the art and, specifically, the amino acids can be branched-chain amino acids (BCAA). The term "BCAA" refers to amino acids that have a central carbon atom bonded to three or more carbon atoms, possessing a branched-chain and an aliphatic side chain. Specifically, BCAA may include leucine, valine, or isoleucine.

The monosaccharide and amino acid conjugate compounds according to the present disclosure refer to compounds by combining aforementioned monosaccharides and amino acids in the manner well known in the art. By way of example, the monosaccharide and amino acid conjugate compound may be a compound where BCAA is bonded to fructose. Specifically, the monosaccharide and amino acid conjugate compound could be a compound where leucine, valine, or isoleucine is bonded to fructose, and more specifically, the monosaccharide and amino acid conjugate compound may be represented by the chemical formulas 1 to 3 : 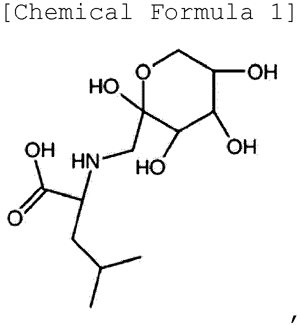 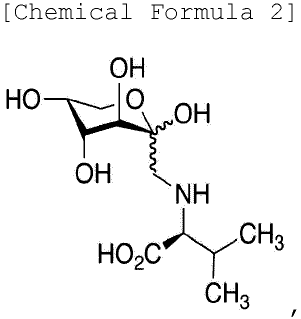 and

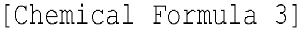

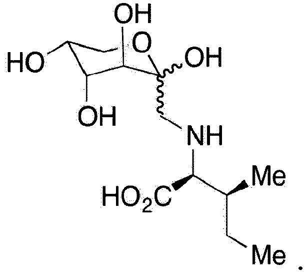

Furthermore, the present disclosure provides a pharmaceutical composition including a monosaccharide and amino acid conjugate compound and a pharmaceutically acceptable salt thereof.

As used herein, the "pharmaceutically acceptable salt" means a salt suitable for use in contact with human and lower animal tissues without causing excessive toxicity, irritation, allergic reaction, and the like within the scope of sound medical judgment. The pharmaceutically acceptable salts are well known in the art and are described. As for details, reference may be made to, for example, "S. M. Berge et al., J. Parmaceutical Sciences, 66, 1, 1977." The salt may be prepared in situ during final isolation and purification of the compound of the present disclosure, or may be prepared by separately reacting with an inorganic base or an organic base. Preferred examples of the base addition salt form may include ammonium salts; alkali salts and alkaline earth metal salts such as salts of lithium, sodium, potassium, magnesium, calcium, and the like; salts with organic bases, for example, primary, secondary and tertiary aliphatic and aromatic amines such as methylamine, ethylamine, propylamine, isopropylamine, four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline; benzathine, N-methyl-D-glucamine, 2-amino (hydroxymethyl)-1,3-propanediol, hydrabamine salt; and salts with amino acids such as arginine, lysine and the like.

Additionally, the present disclosure may include hydrates or solvates of the monosaccharide and amino acid conjugate compound, as well as derivative compounds thereof. The solvents included for these solvates are not particularly limited and may include any conventional solvents known in the field.

The muscle disease may be due to muscle loss. In another aspect, the muscle disease could be a progressive condition characterized by a gradual decrease in muscle strength, resulting in the loss of mobility, weakening of respiratory muscles, and weakening of cardiac function. Moreover, the muscle disease may be congenital or acquired. Examples of diseases caused by muscle reduction include atony, muscular atrophy, muscular dystrophy, muscle degeneration, myotonia, muscular dystrophy, amyotrophic lateral sclerosis (ALS), myasthenia, cachexia, or sarcopenia.

The pharmaceutical composition according to the present disclosure may include the monosaccharide and amino acid conjugate compound or pharmaceutically acceptable thereof as an active ingredient in an amount of 10 to 95 % by weight based on the total weight thereof. The pharmaceutical composition of the present disclosure may include, in addition to the active ingredient, one or more effective ingredients having identical or similar function to the active ingredient.

The pharmaceutical composition of the present disclosure may include carriers, diluents, excipients or mixtures thereof generally used in biological preparations. The pharmaceutically acceptable carrier may be any carrier suitable for delivering the composition in human body. In detail, the carrier may be saline, sterilized water, Ringer's solution, dextrose solution, maltodextrin solution, glycerol, ethanol, or a mixture thereof, as exemplified by the compounds described in Merck Index, 13th ed., Merck & Co. Inc. If necessary, a general additive such as antioxidant, buffer, bacteriostatic agent and others may be additionally added.

When formulating the composition, commonly used diluents or excipients, such as fillers, extenders, binders, wetting agents, disintegrants, and surfactants, can be added.

The composition of the present disclosure can be formulated as oral preparations or non-oral preparations. Oral formulations can include solid formulations and liquid formulations. The solid formulations may be tablets, pills, powders, granules, capsules or troches. These solid formulations are prepared by mixing the composition with one or more suitable excipients. These excipients may include starch, calcium carbonate, sucrose, lactose, gelatin, or a mixture thereof. Furthermore, the solid preparation may contain lubricants such as magnesium stearate and talc. The liquid formulations can be suspensions, solutions, emulsions or syrups, and the above-mentioned liquid formulations can contain various excipients such as wetting agents, sweeteners, aromatics and preservatives.

Formulations for non-oral administration can include injections, suppositories, powders for respiratory inhalation, aerosols for spray, powders and creams. The injection can include sterilized aqueous solutions, non-aqueous solvents, suspension solvent, emulsions, and the like. In this regard, examples of the non-aqueous solvent or suspension solvent include propylene glycol, polyethylene glycol, vegetable oil such as olive oil, injectable ester such as ethyl oleate, and the like.

Additionally, the present disclosure provides a health functional food including a monosaccharide and amino acid conjugate compound, or a pharmaceutically acceptable salt thereof as an active ingredient for the prevention or alleviation of muscle diseases.

The monosaccharide and amino acid conjugate compound or pharmaceutically acceptable salt thereof included in the health functional food according to the present disclosure may possess the characteristics as previously described. The muscle diseases addressed may also have the characteristics as outlined in the foregoing.

The monosaccharide and amino acid conjugate compound or pharmaceutically acceptable salt thereof of the present disclosure can be added directly to food or used in conjunction with other food ingredients. The content of the active ingredient added may be determined according to the purpose and typically may constitute 0.01 to 90 weight parts based on the total weight of the health functional food.

No particular limitations are limited to the form and type of the health functional food. Specifically, the health functional food may be in the form of tablets, capsules, powders, granules, liquids, or pills. The health functional food may include various additives such as flavorings, sweeteners, or natural carbohydrates. The sweeteners may be natural or synthetic; examples of natural sweeteners include thaumatin, stevia extract, and others, while examples of synthetic sweeteners include saccharin, aspartame, and others. Additionally, the natural carbohydrates may include monosaccharides, disaccharides, polysaccharides, oligosaccharides, sugar alcohols, and others.

The health functional food of the present disclosure, in addition to the aforementioned additives, may further include nutrients, vitamins, electrolytes, flavor enhancers, colorants, pectic acid and its salts, alginic acid and its salts, organic acids, protective colloidal thickening agents, pH adjusters, stabilizers, preservatives, glycerin, alcohol, and others. These components may be used independently or in combination. The ratio of these additives may be selected within the range of 0.01 to 0.1 weight parts per 100 weight parts of the composition of the present disclosure.

Additionally, the present disclosure provides a health functional food including a monosaccharide and amino acid conjugate compound, or a pharmaceutically acceptable salt thereof as an active ingredient for muscle strengthening.

The monosaccharide and amino acid conjugate compound d or pharmaceutically acceptable salt thereof included in the health functional food according to the present disclosure may possess the characteristics as previously described. The health functional food itself may also have the characteristics as outlined above.

Furthermore, the present disclosure provides a method for the prevention, alleviation, or treatment of muscle diseases, the method including a step of administering a monosaccharide and amino acid conjugate compound, or a pharmaceutically acceptable salt thereof to a subject.

The monosaccharide and amino acid conjugate compound or pharmaceutically acceptable salt thereof used in the method according to the present disclosure can have the characteristics as described above. The muscle diseases addressed may also have the characteristics as previously mentioned.

The subject may be a mammal, specifically a human.

Depending on a desired manner, the administration may be oral or non-oral, where non-oral administration may include intraperitoneal, rectal, subcutaneous, intravenous, intramuscular, or intrathoracic injection methods.

The administration should be in a pharmacologically effective amount, which can vary depending on the type of disorder, its severity, the activity of the drug, patient sensitivity to the medication, timing of administration, route of administration, duration of treatment, and any concurrently used medications. However, for desired effects, the amount of the active ingredient included in the administration can range from 0.0001 to 1,000 mg/kg, specifically 0.001 to 500 mg/kg. The administration may occur once or several times a day.

The administration can be performed alone or in combination with other therapeutic agents. When combined, the administration may be sequential or simultaneous.

Moreover, the present disclosure provides a use of the monosaccharide and amino acid conjugate compound or pharmaceutically acceptable salt thereof in preparing medicaments for the prevention, alleviation, or treatment of muscle diseases.

The elderberry extract used in the preparation of the medicaments according to the present disclosure may have the characteristics as described above. The muscle diseases addressed may also have the same characteristics.

### Mode for Carrying Out the Invention

Hereinafter, the present disclosure will be described in detail by the following examples, provided that the following examples are only for illustrating the present disclosure, and the present disclosure is not limited by them. Any is included in the technical scope of the present disclosure if it has substantially the same constitution as the technical idea described in the claim of the present disclosure and offers similar operation and effect thereto.

### EXAMPLE 1. Preparation of Elderberry Hot Water Extract

A hot water extract from dried elderberries was prepared as follows.

First, 10-fold weight purified water was added to the dried elderberries, followed by conducting extraction at 80°C for 3 hours to obtain an extract. The obtained extract was filtered using a metal detector and concentrated to give an elderberry concentrate. The elderberry concentrate was mixed with 30% maltodextrin and freeze-dried to afford a powder of the hot water extract of elderberry.

### EXAMPLE 2. Preparation of 50% Ethanol Extract of Elderberry

A powder of 50% ethanol extract of elderberry was prepared in the same manner and condition as in Example 1, with the exception of adding 50% ethanol instead of purified water and conducting extraction for 6 hours.

### EXAMPLE 3. Preparation of 100% Ethanol Extract of Elderberry

A powder of 100% ethanol extract of elderberry was prepared in the same manner and condition as in Example 1, with the exception of adding 100% ethanol instead of purified water and conducting extraction for 6 hours.

### EXAMPLE 4. Analysis of Components in Elderberry Extracts

The hot water extract of elderberry prepared above was analyzed using liquid chromatography/mass spectrometry (LC/MS) to detect fructose-leucine (FL) within the extract.

Specifically, 200 mg of the hot water extract powder of elderberry was dissolved in water and added with methanol, and the mixture was ultrasonically treated for more than 60 minutes to prepare the sample. Meanwhile, a calibration curve was created using 0.2 mg/L of FL to set the quantification range. The prepared samples were analyzed under these conditions, and the analysis was repeated in triplicate. The content of FL in the hot water extract of elderberry was calculated using the obtained peak area and retention time (RT), and the results are shown in Table 1.

**TABLE 1**

| Sample | Peak Area | Specimen RT | FL Content (%) | Average FL Content (%) |
|---|---|---|---|---|
| #1 | 7844 | 2.899 | 1.02 | 1.02 |
| #2 | 7713 | 2.892 | 1.01 | |
| #3 | 7869 | 2.904 | 1.03 | |

As shown in Table 1, the content of FL in the samples was found to be 1.02% on average.

### EXPERIMENTAL EXAMPLE 1: Inhibition of Myocyte Apoptosis

An examination was made to determine whether the elderberry extracts and monosaccharide-amino acid conjugate compounds prepared above inhibit myocyte apoptosis, as follows:

### 1-1. Preparation of Myotubes

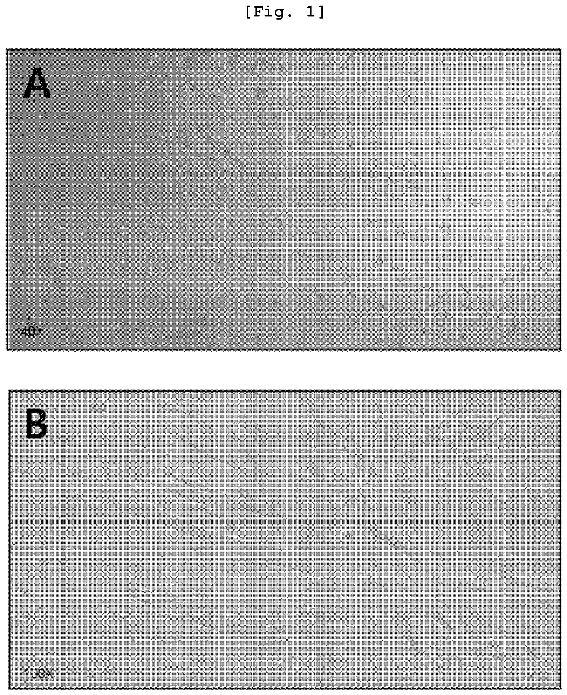

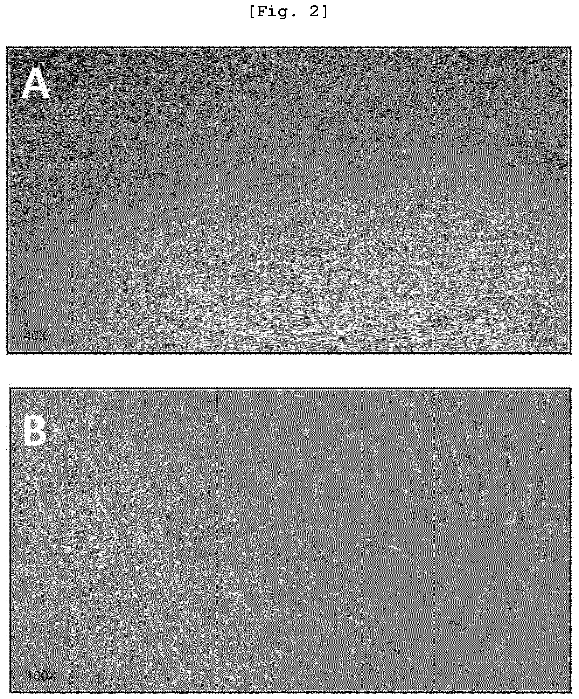

Firstly, C2C12 myoblast cells (from ATCC, USA) were cultured in DMEM supplemented with 10% fetal bovine serum (FBS) and 1% antibiotic-antimycotic at 37°C under a 5% CO2 atmosphere. Thereafter, the medium was replaced with one containing 2% horse serum (HS) for incubation of additional 72 hours. The cells were observed under a microscope to confirm differentiation from myoblasts to myotubes, and then treated with 10 µM dexamethasone for 24 hours. Post-treatment, the cells were observed again under a microscope. The results of the differentiation to myotubes are presented in FIG. 1, and the observations post-dexamethasone treatment in FIG. 2.

As shown in FIG. 1, myoblasts had differentiated into myotubes. Furthermore, as depicted in FIG. 2, the treatment with dexamethasone resulted in thinner myotubes, indicating muscle reduction.

### 1-2. Inhibition of Myocyte Apoptosis by Elderberry Extract

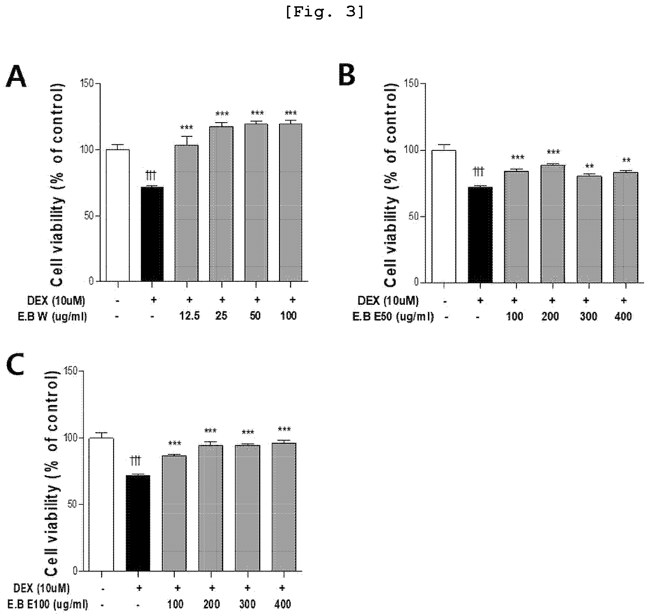

The cell line treated with dexamethasone was then treated with varying concentrations of the hot water extract of elderberry (12.5, 25, 50, or 100 pg/mL), the 50% ethanol extract of elderberry (100, 200, 300, or 400 pg/mL), or the 100% ethanol extract of elderberry (100, 200, 300, or 400 pg/mL) . After 24 hours, the culture medium was removed, and MTT reagent (5 mg/mL) was added to each well and incubated for 4 hours. Subsequently, DMSO was added to dissolve any precipitates, and the absorbance was read at 570 nm. Cell viability was calculated by a typical method using the measured values and the results are shown in FIG. 3.

As indicated in FIG. 3, cell viability reduced by dexamethasone was significantly restored by treatment with elderberry extracts.

### 1-3. Inhibition of Myocyte Apoptosis by Monosaccharide and Amino Acid Conjugate Compounds

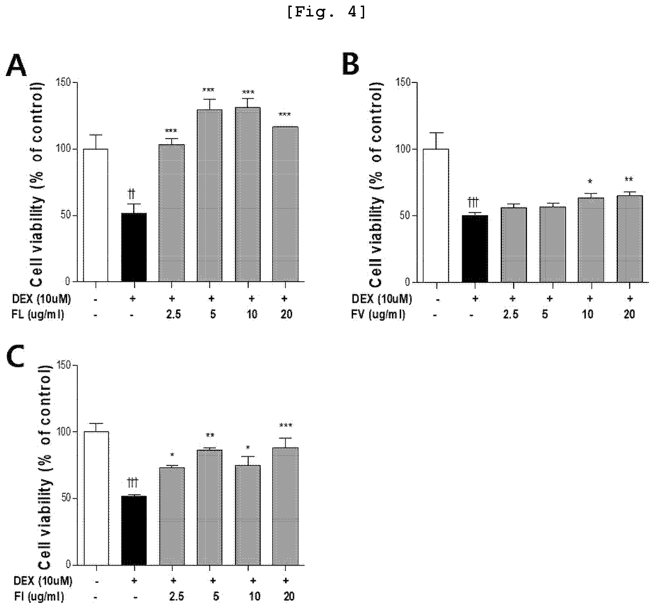

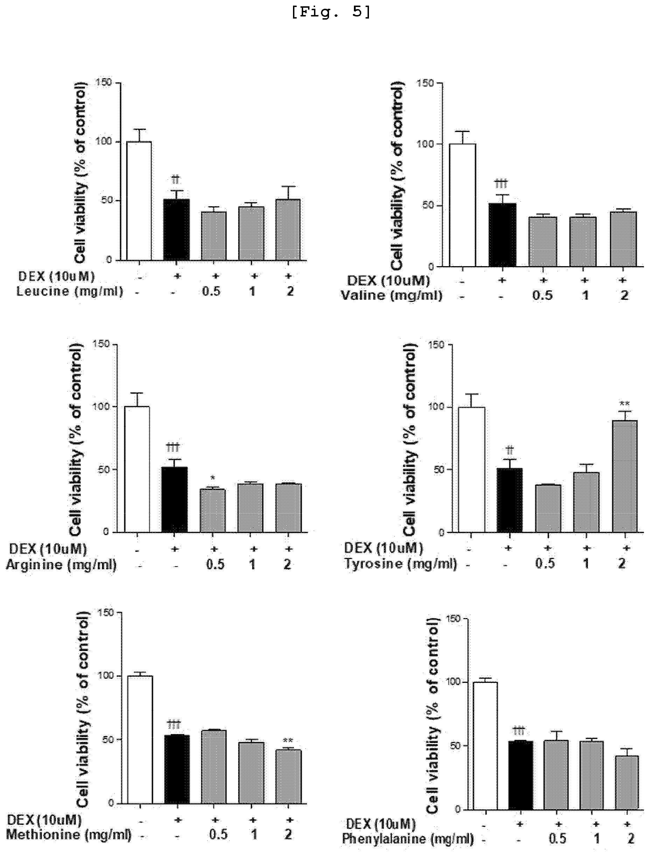

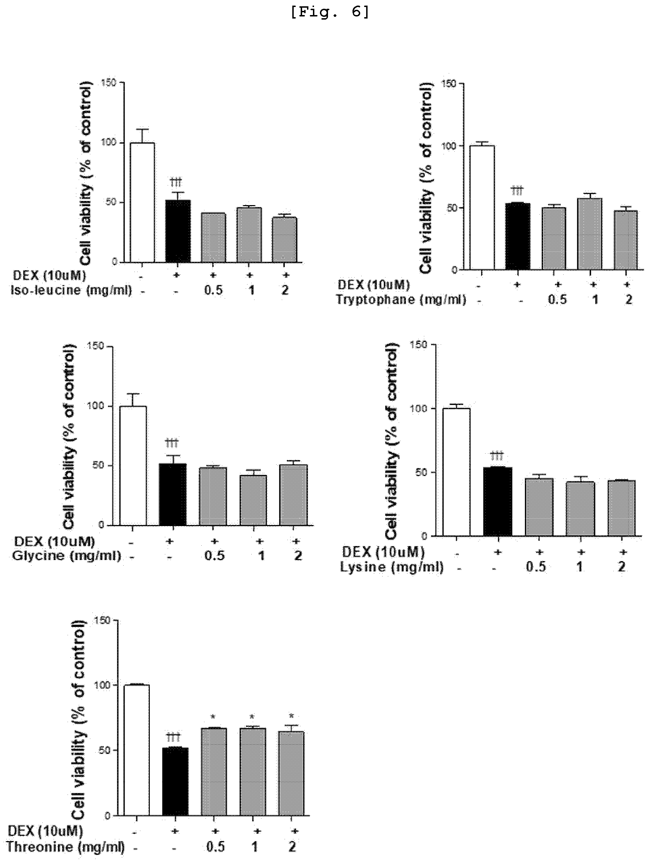

Apart from FL, which was found in elderberry extracts, fructose-valine (FV) and fructose-isoleucine (FI) conjugates in which the branched-chain amino acid valine or isoleucine is conjugated to the monosaccharide fructose were also tested for inhibition against myocyte apoptosis. Experiments were conducted in the same manner and conditions as in Experiment Example 1-2, except that FL, FV, or FI was used at concentrations of 2.5, 5, 10, or 20 µg/mL instead of elderberry extracts. As controls, amino acids such as leucine, valine, isoleucine, tryptophan, arginine, tyrosine, glycine, lysine, methionine, phenylalanine, or threonine were used. The cell viability calculated from the treatments with FL, FV, or FI are presented in FIG. 4, and results from amino acid treatments in FIGS. 5 and 6.

As shown in FIG. 4, cell viability reduced by dexamethasone was significantly restored by FL, FV, or FI treatments. In contrast, as shown in FIGS. 5 and 6, treatments with amino acids did not restore cell viability.

### EXPERIMENTAL EXAMPLE 2: Inhibition of Inflammatory Cytokine Expression

The elderberry extracts and monosaccharide-BCAA conjugate compounds prepared above were examined for ability to inhibit the expression of inflammatory cytokines, as follows.

### 2-1. Inhibition of TNFα Expression by Elderberry Extract

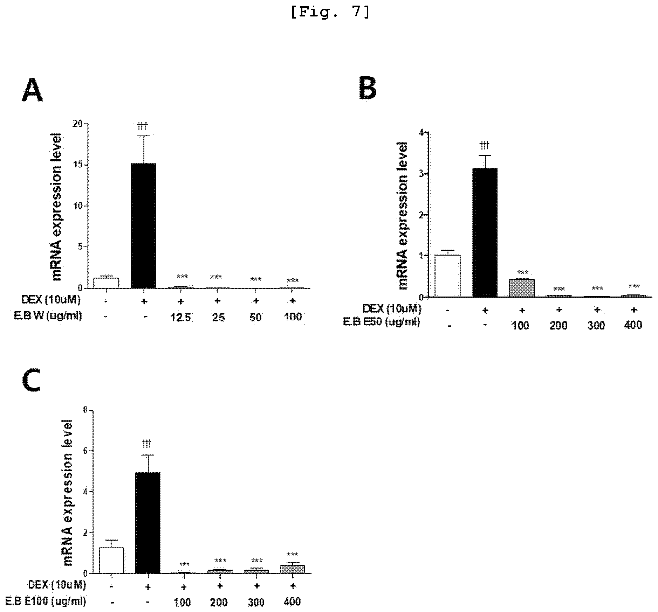

The cells treated with elderberry extracts as in Experimental Example 1-2 were further treated with trypsin to collect only the cells. After centrifugation of the collected cells at 3,000 rpm for 5 minutes, the supernatant was removed and total RNA was extracted by sequentially treating with RiboEx^{™} and Hybrid-R^{™} (GeneAll, Korea) . The concentration of the extracted RNA was measured using a Nanodrop, and this RNA was used as a template to synthesize cDNA. The cDNA was synthesized using a mixture of RNA, oligo-(dT) primers, and 2×HyperScript^{™} RT Master Mix (GeneAll, Korea). Subsequently, real-time PCR (qPCR) was performed using the synthesized cDNA as a template and Power SYBR^{™} Green PCR Master Mix under standard conditions to confirm the expression of TNFα. The results were graphically presented in FIG. 7.

As shown in FIG. 7, the level of TNFα expression, which was increased by dexamethasone, was significantly suppressed by treatment with elderberry extracts.

### 2-2. Inhibition of TNFα Expression by Monosaccharide and Amino Acid Conjugate Compounds

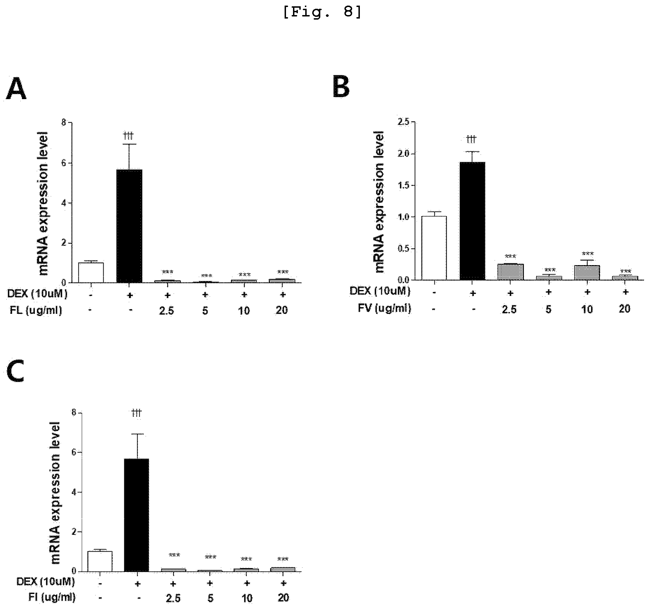

Cells treated with FL, FV, or FI as in Experimental Example 1-3 were trypsinized to collect only the cells, and the level of TNFα expression was examined using the same conditions and methods as described in Experimental Example 2-1. The results were graphically presented in FIG. 8.

As shown in FIG. 8, the level of TNFα expression, which was increased by dexamethasone, was significantly suppressed by the treatment with FL, FV, or FI.

Therefore, it was understood from these results that elderberry extracts, and monosaccharide and amino acid conjugate compounds significantly inhibited the expression of inflammatory cytokines, which directly induce sarcopenia, and thus suppressed the myocyte apoptosis induced by inflammatory cytokines.

### EXPERIMENTAL EXAMPLE 3: Promotion of Male Hormone Secretion

The ability of the elderberry extracts and monosaccharide-BCAA conjugate compounds prepared above to inhibit the muscle loss induced by the male hormone testosterone was tested using the ELISA analysis method as follows:

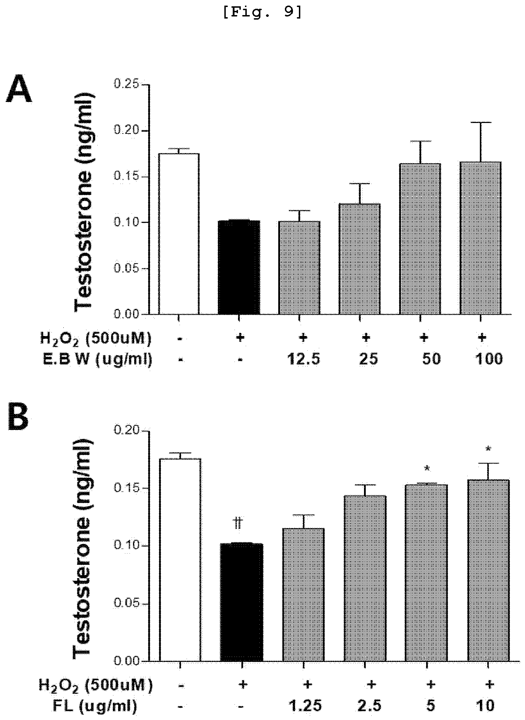

First, the TM3 cell line (from ATCC, USA), which is Leydig cells, were cultured using a standard method. The cultured cells were treated with 500 µM of hydrogen peroxide (H₂O₂) for 6 hours, followed by the addition of either 12.5, 25, 50, or 100 ug/mL of the hot water extract of elderberry or 1.25, 2.5, 5, or 10 ug/mL of FL (fructose-leucine). Subsequently, ELISA was performed using the cultured cell medium to determine the levels of testosterone, and the results are presented in FIG. 9.

As shown in FIG. 9, the concentration of testosterone, which was suppressed by hydrogen peroxide, was significantly restored by treatment with either the hot water extract of elderberry, or FL.

### EXPERIMENTAL EXAMPLE 4: Inhibition of Muscle Protein Expression

The ability of the elderberry extracts and monosaccharide-BCAA conjugate compounds prepared above to inhibit the expression of muscle proteins was confirmed using the qPCR method. Specifically, muscle RING-finger protein-1 (MuRF-1) and atrogin-1, which are both markers of muscle atrophy, and myostatin, which a negative regulator of skeletal muscle mass, were used as target muscle proteins.

### 4-1. Inhibition of Muscle Protein Expression by Elderberry Extracts

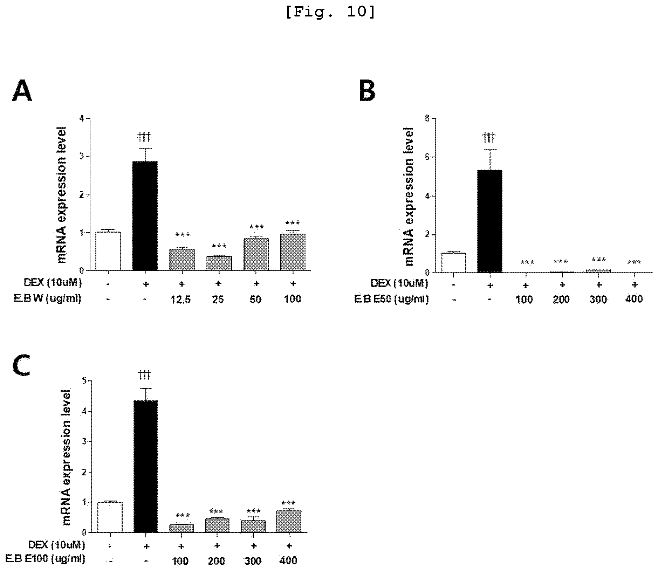

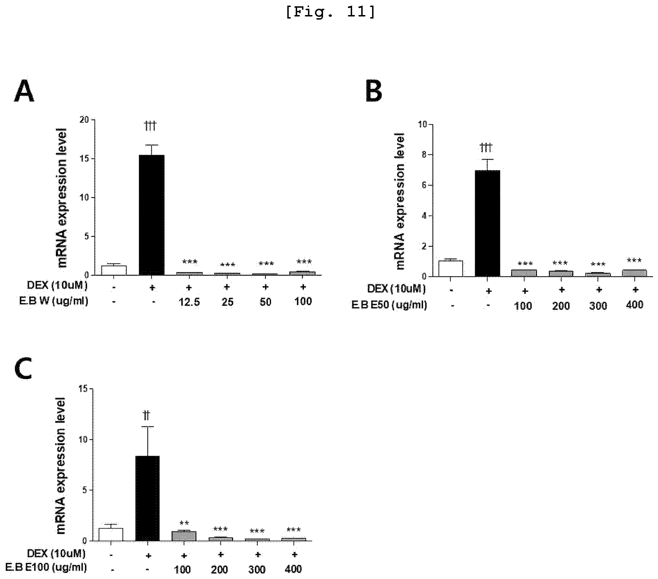

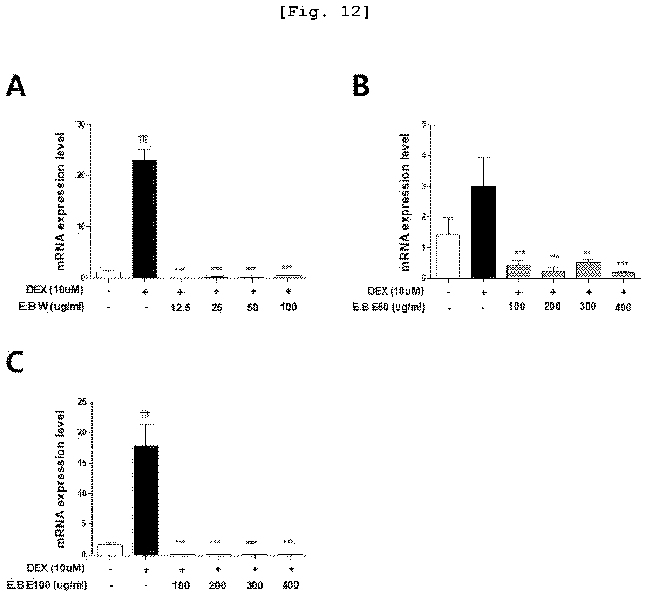

Using the cells treated with the hot water extract, 50% ethanol extract, or 100% ethanol extract of elderberry described in Experimental Example 1-2, qPCR was performed as described above. The results showed the levels of mRNA expression for MuRF-1, atrogin-1, and myostatin mRNA, which are depicted in FIGS. 10 to 12.

As shown in FIGS. 10 to 12, the mRNA expression levels of MuRF-1, atrogin-1, and myostatin, which were increased by dexamethasone, were significantly decreased by the treatment with elderberry extracts.

### 4-2. Inhibition of Muscle Protein Expression by Monosaccharide and Amino Acid Conjugate Compounds

Using the cells treated with FL, FV, or FI as described in Experimental Example 1-3, qPCR was performed as previously outlined. The results showed the levels of mRNA expression for MuRF-1, atrogin-1, and myostatin mRNA, which are depicted in FIGS. 10 to 12.

As shown in FIGS. 10 to 12, the mRNA expression levels of MuRF-1, atrogin-1, and myostatin, which were increased by dexamethasone, were significantly decreased by the treatment with FL, FV, or FI.

Therefore, these results indicate that both elderberry extracts and monosaccharide-BCAA conjugate compounds can exhibit therapeutic effects in the treatment of sarcopenia by inhibiting the expression of muscle atrophy markers and myostatin.

### EXPERIMENTAL EXAMPLE 5: Effectiveness in Sarcopenia Animal Model

The therapeutic effects of the elderberry extracts and monosaccharide-BCAA conjugate compounds on sarcopenia were evaluated using a sarcopenia animal model.

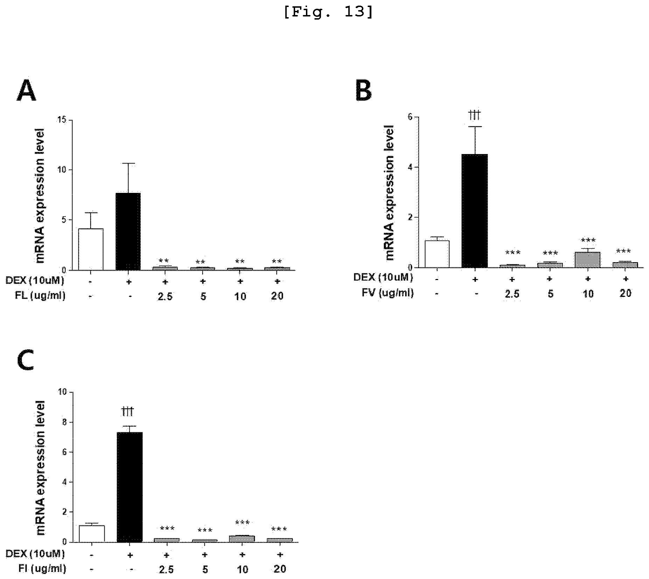

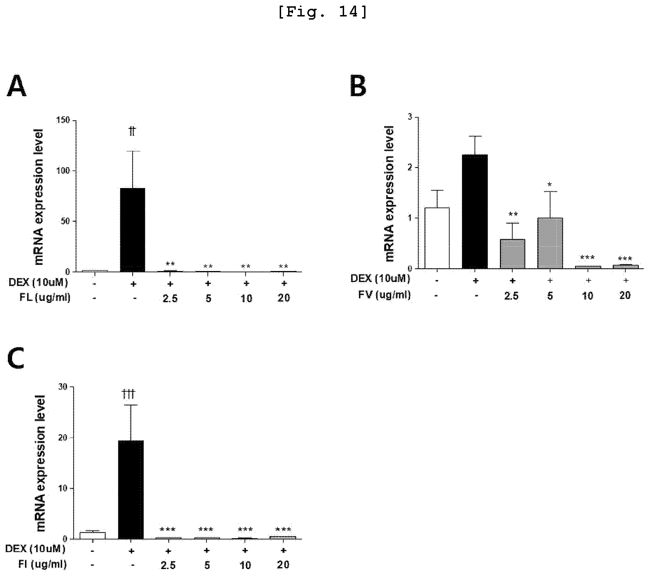

Specifically, male C57BL/6 mice (7 weeks old, Samtako Bio Korea, Korea) were acclimatized, and their body weight (g), lean body weight (g), fat mass (g), and body fat percentage (%) were measured using dual-energy X-ray absorptiometry (DXA). Considering these measurements, the mice were grouped into sets of seven, and sarcopenia was induced by administering 20 mg/kg of dexamethasone intraperitoneally once daily for 14 days (FIG. 13). Simultaneously with the dexamethasone treatment, 300 mg/kg of hot water extract of elderberry or 0.5 mg/kg of FL was administered orally once daily. A control group consisted of untreated mice (normal mice) at day 0 of treatment. On days 7 and 14 of treatment, the mice were scanned using DXA to measure body weight, lean body weight, fat mass, and body fat percentage; the results are presented in FIG. 14 and Tables 2 and 3. Additionally, on day 14, the mice were sacrificed using standard methods, and the thymus, spleen, liver, and muscle were excised and weighed; these results are summarized in Table 4.

**TABLE 2**

| | | Normal Control | Dexamethasone administered | Hot water extract of elderberry administered |
|---|---|---|---|---|
| Day 0 | lean body weight (g) | 18.84±1.03 | 18.93±1.42 | 18.6510.71 |
| | fat (g) | 1.28±0.16 | 1.44±0.16 | 1.410.19 |
| | fat (%) | 6.3710.79 | 7.1±1.07 | 6.98±1.00 |
| | tissue area (cm²) | 13.7110.62 | 13.3212.03 | 13.98±0.84 |
| | BMD(g/cm²) | 0.053±0.003 | 0.057±0.012 | 0.055±0.004 |
| | BMC (g) | 0.18±0.02 | 0.2310.13 | 0.1810.02 |
| Day 7 | lean body weight (g) | 21.14±1.32 | 17.53±1.36 | 17.79±1.71 |
| | fat (g) | 1.60±0.43 | 1.13±0.34 | 1.68±0.57 |
| | fat (%) | 6.98±1.61 | 5.99±1.53 | 8.5±2.3 |
| | tissue area(cm²) | 13.69±1.27 | 12.41±1.31 | 13.88±1.17 |
| | BMD(g/cm²) | 0.057±0.007 | 0.052±0.004 | 0.054±0.007 |
| | BMC (g) | 0.26±0.08 | 0.16+0.02 | 0.19±0.04 |
| Day 14 | lean body weight (g) | 22.47±0.59 | 17.47±0.29 | 19.22±0.5 |
| | fat (g) | 1.65±0.39 | 1.56±0.34 | 1.79±0.45 |
| | fat (%) | 6.79±1.37 | 8.17±1.58 | 8.49±1.94 |
| | tissue area(cm²) | 15.12±1.01 | 13.97±0.51 | 15.29±0.50 |
| | BMD(g/cm²) | 0.064±0.004 | 0.057±0.004 | 0.058±0.002 |
| | BMC (g) | 0.29±0.09 | 0.16±0.01 | 0.18±0.01 |

**TABLE 3**

| | | Normal Control | Dexamethasone administered | FL administered |
|---|---|---|---|---|
| Day 0 | lean body weight (g) | 18.84±1.03 | 18.93±1.42 | 18.31±1.43 |
| | fat (g) | 1.28±0.16 | 1.44±0.16 | 1.27±0.25 |
| | fat(%) | 6.37±0.79 | 7.11±.07 | 6.49±1.03 |
| | tissue area(cm²) | 13.71±0.62 | 13.32±2.03 | 13.13±1.08 |
| | BMD (g/cm²) | 0.053±0.003 | 0.057±0.012 | 0.053±0.004 |
| | BMC (g) | 0.18±0.02 | 0.23±0.13 | 0.19±0.04 |
| Day 7 | lean body weight (g) | 21.14±1.32 | 17.53±1.36 | 18.05±0.44 |
| | fat (g) | 1.60±0.43 | 1.13±0.34 | 1.43±0.53 |
| | fat(%) | 6.98±1.61 | 5.99±1.53 | 7.31±2.47 |
| | tissue area (cm²) | 13.69±1.27 | 12.41±1.31 | 13.83±0.53 |
| | BMD(g/cm²) | 0.057±0.007 | 0.052±0.004 | 0.059±0.009 |
| | BMC (g) | 0.26±0.08 | 0.16±0.02 | 0.20±0.03 |
| Day 14 | lean body weight (g) | 22.47±0.59 | 17.47±0.29 | 18.34±0.56 |
| | fat (g) | 1.65±0.39 | 1.56±0.34 | 1.29±0.19 |
| | fat(%) | 6.79±1.37 | 8.17±1.58 | 6.57±0.76 |
| | tissue area(cm²) | 15.12±1.01 | 13.97±0.51 | 13.66±0.92 |
| | BMD(g/cm²) | 0.064±0.004 | 0.057±0.004 | 0.056±0.006 |
| | BMC(g) | 0.29±0.09 | 0.16±0.01 | 0.19±0.04 |

**TABLE 4**

| Tissue | Normal Control | Dexamethas one administer ed | Hot water extract of elderberry administered | FL administere d |
|---|---|---|---|---|
| Thymus (g) | 0.036±0.00 6 | 0.009±0.00 4 | 0.011±0.003 | 0.009±0.002 |
| Spleen (g) | 0.075±0.00 7 | 0.026±0.01 | 0.026±0.009 | 0.028±0.009 |
| Liver (g) | 1.338±0.10 6 | 1.514±0.13 4 | 1.661±0.302 | 1.375±0.117 |
| Muscle (g) | 0.608±0.04 5 | 0.501±0.06 4 | 0.598±0.142 | 0.683±0.18 |

As shown in FIG. 14 and Tables 2 and 3, the body weight, lean body weight, and tissue areas of the mice significantly decreased due to the dexamethasone, but these were restored by the administration of hot water extract of elderberry or FL. Moreover, the weights of the various tissues also decreased due to the dexamethasone, but were restored to levels similar to those of the normal control group by the administration of the hot water extract of elderberry or FL.

Therefore, these results indicate that the elderberry extract, as well as the monosaccharide and amino acid conjugate compounds, significantly alleviate sarcopenia, demonstrating their therapeutic efficacy in treating sarcopenia syndrome.

## Claims

1. A pharmaceutical composition for prevention or treatment of a muscle disease, the composition comprising an elderberry extract as an active ingredient.

2. The pharmaceutical composition of claim 1, wherein the extract is obtained using water, a lower alcohol of C₁ to C₂, or a mixture thereof.

3. The pharmaceutical composition of claim 2, wherein the lower alcohol of C₁ to C₂ is ethanol, methanol, or rectified spirit.

4. The pharmaceutical composition of claim 1, wherein the muscle disease is a disease caused by muscle loss.

5. The pharmaceutical composition of claim 4, wherein the disease caused by muscle loss is atony, muscular atrophy, muscular dystrophy, muscle degeneration, myotonia, muscular dystrophy, amyotrophic lateral sclerosis (ALS), myasthenia, cachexia, or sarcopenia.

6. A health functional food for prevention or alleviation of a muscle disease, the health function food comprising an elderberry extract.

7. A health functional food for muscle strengthening, the health functional food comprising an elderberry extract as an active ingredient.

8. A method for prevention, alleviation, or treatment of a muscle disease, the method comprising a step of administering an elderberry extract into a subject.

9. Use of an elderberry extract for preparing a medicament for prevention, alleviation, or treatment of a muscle disease.

10. A pharmaceutical composition for prevention or treatment of a muscle disease, the pharmaceutical composition comprising a monosaccharide and amino acid conjugate compound, or a pharmaceutically acceptable salt thereof as an active ingredient.

11. The pharmaceutical composition of claim 10, wherein the monosaccharide is fructose.

12. The pharmaceutical composition of claim 10, wherein the amino acid is a branched-chain amino acid (BCAA).

13. The pharmaceutical composition of claim 12, wherein BCAA is leucine, valine, or isoleucine.

14. The pharmaceutical composition of claim 10, wherein the monosaccharide and amino acid conjugate compound is a compound represented by one of the following Chemical Formulas 1 to 3: 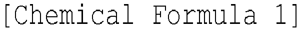 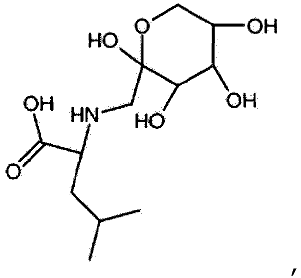 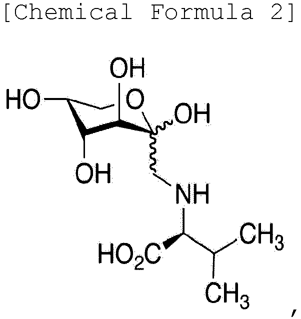 and

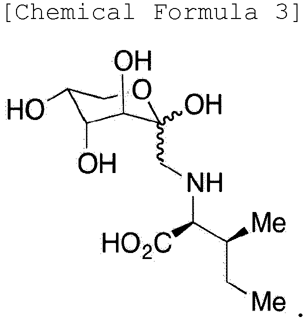

15. The pharmaceutical composition of claim 10, wherein the muscle disease is a disease caused by muscle loss.

16. The pharmaceutical composition of claim 15, wherein the disease caused by muscle loss is atony, muscular atrophy, muscular dystrophy, muscle degeneration, myotonia, muscular dystrophy, amyotrophic lateral sclerosis (ALS), myasthenia, cachexia, or sarcopenia.

17. A health functional food for prevention or alleviation of a muscle disease, the health functional food comprising a monosaccharide and amino acid conjugate compound and a pharmaceutically acceptable salt thereof as an active ingredient.

18. A health functional food for muscle strengthening, the health functional food comprising a monosaccharide and amino acid conjugate compound and a pharmaceutically acceptable salt thereof as an active ingredient.

19. A method for prevention, alleviation, or treatment of a muscle disease, the method comprising a step of administering a monosaccharide and amino acid conjugate compound, or a pharmaceutically acceptable salt thereof as an active ingredient.

20. Use of a monosaccharide and amino acid conjugate compound, or a pharmaceutically acceptable salt thereof for preparing a medicament for prevention, alleviation, or treatment of a muscle disease.
